# FASCICULE DE BREVET EUROPEEN

(11) **EP 3 829 660 B1**
(45) Date de publication et mention de la délivrance du brevet: **30.09.2026**
(21) Numéro de dépôt: 19745139.6
(22) Date de dépôt: 26.07.2019
(51) Int. Cl.: A61L 9/12

(54) **CARTOUCHE POUR UN DISPOSITIF DE DIFFUSION DE FRAGRANCES ET DISPOSITIF POUR LA DIFFUSION DE FRAGRANCES**
KARTUSCHE FÜR EINEN DUFTSPENDER UND DUFTSPENDER
CARTRIDGE FOR A FRAGRANCE DISPENSING DEVICE AND FRAGRANCE DISPENSING DEVICE

(30) Priorité: 27.07.2018 FR 1857018
(43) Date de publication de la demande: 09.06.2021
(73) Titulaire: Artiris, 75017 Paris (FR)
(72) Inventeur: WUIDART, Aymeric, 75017 PARIS (FR); LOPEZ-BONET, David, 75017 PARIS (FR)
(74) Mandataire: Jacobacci & Partners France
(86) Numéro de dépôt international: PCT/EP2019/070201
(87) Numéro de publication internationale: WO 2020/021076

(56) Documents cités:
- WO-A1-2018/091766
- JP-A- 2018 086 230
- KR-Y1- 200 438 243

## Description

La présente invention concerne le domaine technique des dispositifs de diffusion de fragrances, et plus particulièrement les diffuseurs comprenant des cartouches de fragrances ou senteurs. L'invention concerne également une cartouche pour un dispositif de diffusion de fragrances.

Dans le domaine ci-dessus, il est connu des diffuseurs de parfums à diffusion sèches comprenant des cartouches, montées de manière amovible dans un diffuseur, aptes à libérer au moins une fragrance.

Par exemple, le document WO2016/098114 divulgue un système de diffusion de fragrances comprenant un boitier avec des logements ouverts à l'air libre, chacun destiné à recevoir une cartouche renfermant une fragrance. Selon ce document, chaque cartouche est constituée d'un corps creux cylindrique qui renferme un réservoir de fragrance et dont les faces haute et basse sont perforées de manière à permettre le passage d'un flux d'air au contact du réservoir. Chaque logement est, en outre, associé à un ventilateur adapté pour pulser de l'air dans le logement à partir de sa base et donc au travers de la cartouche qui s'y trouve. Le système diffuse alors les fragrances en modulant la vitesse de fonctionnement des ventilateurs selon des commandes de l'utilisateur.

Un tel système permet la diffusion de fragrances en étant facile d'utilisation mais présente notamment l'inconvénient de laisser le réservoir de chaque cartouche toujours au contact de l'air ambiant, de sorte que les fragrances sont diffusées en permanence, même en cas d'arrêt du ventilateur associé à la cartouche ou d'arrêt complet du dispositif. Il en résulte un vidage incontrôlé du réservoir des cartouches et l'impossibilité d'assurer une composition olfactive qui ne comprendrait pas la fragrance d'une certaine cartouche, dans la mesure où même avec son ventilateur associé arrêté, cette dernière diffuse toujours les principes aromatiques qu'elle contient. Autres systèmes de diffusion sont connus de WO 2018/091766 A1, KR 200 438 243 Y1 et JP 2018 086230 A.

Afin de remédier à cet inconvénient, l'invention concerne un dispositif selon la revendication 1 et une cartouche selon la revendication 5. La présente divulgation comprend également des modes de réalisation qui ne relèvent pas du champ d'application de l'invention défini dans les revendications.

Ainsi, l'intégration à la cartouche de moyens d'obturation étanches à l'air asservis en fermeture évite un vidage ou une altération du contenu de la cartouche lorsqu'elle est en dehors de l'appareil. De plus, les moyens d'obturation offrent la possibilité d'obtenir une parfaite fermeture de chaque cartouche dans le dispositif de diffusion lorsque la fragrance contenue dans la cartouche ne doit pas être mise en œuvre. Par étanche à l'air, il convient d'entendre qu'aucune fuite d'air n'intervient lorsque la pression à l'intérieur de la cartouche est légèrement supérieure à la pression externe. Dans ce contexte étanche à l'air est synonyme d'hermétique.

Selon une caractéristique de la divulgation les moyens d'obturation comprennent deux obturateurs, l'un pour l'entrée et l'autre pour la sortie.

Selon une autre caractéristique de la divulgation la cartouche comprend au moins un bouchon comprenant :
- un élément de liaison adapté sur le corps de la cartouche de manière étanche à l'air et comprenant un siège définissant un passage,
- un obturateur, mobile en translation, apte à translater entre une position d'ouverture et une position de fermeture du passage, et
- des moyens d'asservissement en fermeture de l'obturateur.
La mise en œuvre d'un tel bouchon permet de simplifier le processus de fabrication de la cartouche et notamment la mise place dans le corps creux des moyens d'accumulation d'au moins une fragrance ou composition odorante. La cartouche peut ensuite être fermée de manière étanche à l'air aux moyens des bouchons.

Selon une autre caractéristique de la divulgation les moyens d'asservissement en fermeture de l'obturateur comprennent un ressort. L'utilisation d'un ressort ou tout autre organe élastique permet d'asservir simplement l'obturateur en position fermée. Ce ressort peut être, par exemple, un ressort à lame ou un ressort hélicoïdal.

Selon la divulgation les moyens d'obturation ne sont pas nécessairement réalisés sous la forme d'un élément mobile et asservi en fermeture par un organe élastique. Ainsi, les moyens d'obturation peuvent être réalisés sous la forme d'une membrane élastiquement déformable avec au moins une fente susceptible de s'ouvrir pour le passage de tube d'admission ou d'échappement d'un flux d'air et refermant automatiquement lors du retrait de ce tube. Lorsqu'elle est fermée la membrane est étanche à l'air.

Selon une autre caractéristique de la divulgation le corps présente, au moins localement, une asymétrie de révolution externe, de manière à définir un sens d'introduction de la cartouche dans un logement, de forme complémentaire, destiné à la recevoir. Cette forme asymétrique assure une fonction de détrompeur évitant une mauvaise mise en place de la cartouche dans le dispositif de diffusion destiné à la recevoir.

Selon une autre caractéristique de la divulgation les moyens d'accumulation d'au moins une composition odorante comprennent un matériau absorbant. La mise en œuvre d'un tel matériau absorbant permet de former un réservoir dans lequel la composition odorante est absorbée de sorte que la manipulation des moyens d'accumulation s'en trouve facilitée notamment pour l'assemblage de la cartouche. En effet, il est alors possible de précharger les moyens d'accumulation avant leur mise en place dans la cartouche.

Selon une autre caractéristique de la divulgation la cartouche comprend un moyen d'identification adapté pour une lecture automatique. La mise œuvre d'un tel moyen permet une identification automatique de chaque cartouche au sein de l'appareil de diffusion qui peut alors modifier son mode de fonctionnement en fonction des informations lues.

La divulgation concerne également un dispositif pour la diffusion de fragrances avec un boîtier comprenant :
- au moins deux logements, chacun adapté pour recevoir, de manière amovible, une cartouche selon l'invention, chaque logement comprenant des moyens d'admission et d'échappement d'un flux d'air qui sont, d'une part, destinés à être raccordés respectivement à l'entrée et à la sortie d'une cartouche disposée dans le logement et, d'autre part, adaptés pour agir sur les moyens d'obturation d'une cartouche située dans le logement de manière à les déplacer en position d'ouverture,
- des moyens de génération de flux d'air, adaptés pour au moins générer des flux d'air traversant les logements entre les moyens d'admission et d'échappement,
- une unité de commande apte à contrôler les moyens de génération de flux d'air et les moyens d'admission et d'échappement et adaptée pour assurer une diffusion indépendante, séquentielle ou simultanée dans des proportions variables des compositions odorantes contenues dans les cartouches et une fermeture des cartouches lors de l'arrêt de l'appareil.

La combinaison des cartouches à obturateurs intégrés asservis en fermeture et des moyens d'admission et d'échappement contrôlés par l'unité de commande permet d'obtenir une modulation précise des mélanges obtenus, dans la mesure où lorsqu'une composition odorante d'une cartouche ne doit pas être mise en œuvre, l'obturation complète de cette dernière permet de garantir qu'aucune émanation de cette dernière ne viendra perturber la diffusion en cours. De plus, la fermeture hermétique des cartouches lors de l'arrêt de l'appareil permet de garantir aucune diffusion intempestive des compositions contenues dans ces dernières, ainsi que de prévenir un vidage prématuré des cartouches.

Par ailleurs, il est apparu à l'usage que le système de diffusion selon l'art antérieur ne permet pas la production d'une modulation de composition olfactive aussi subtile ou précise qu'il aurait pu être attendu par la modulation des flux des ventilateurs. Les inventeurs ont identifié l'ouverture permanente des cartouches comme l'une des causes de ce défaut. Les inventeurs ont également identifié l'imperfection du mélange des fragrances comme une autre cause de l'imperfection de la perception olfactive de la diffusion assurée par le dispositif selon l'art antérieur.

Afin de remédier à cet inconvénient, selon une variante de réalisation de la divulgation le dispositif de diffusion comprend une chambre de mélange qui s'ouvre vers l'extérieur par une bouche de diffusion et à laquelle les moyens d'échappement de chacun des logements sont raccordés en amont de la bouche de diffusion. La mise en œuvre d'une telle chambre de diffusion permet un mélange homogène des différentes compositions olfactives issues des cartouches avant libération à l'air libre ce qui permet de mieux maitriser l'effet ou la perception olfactive obtenus.

Selon une caractéristique de la divulgation la chambre de mélange présente une forme allongée le long d'une fibre moyenne, la bouche de diffusion se trouvant à une extrémité de la chambre de mélange et les moyens d'échappement sont raccordés à la chambre de mélange par des tubulures dont l'axe moyen forme avec la fibre moyenne de la chambre de mélange un angle aigu inférieur à 90 degrés convergeant en direction de la bouche de diffusion. Une telle orientation des tubulures de raccordement permet d'au moins limiter d'éventuels phénomènes de reflux dans les tubulures de raccordement et de réduire les bruits aériens liés à la diffusion.

Selon une variante de cette caractéristique les tubulures de raccordement s'étendent en saillie à l'intérieur de la chambre de mélange. Cette configuration permet d'induire des turbulences dans la chambre de manière à y optimiser le mélange des compositions olfactives.

Au sens de la divulgation un moyen de génération de flux d'air peut être un pulseur d'air tel qu'un ventilateur à hélice ou turbine ou encore un compresseur mécanique tel qu'un compresseur à membrane ou un compresseur à piston. Bien entendu, des moyens de génération de flux d'air peuvent combiner plusieurs systèmes de même type ou de types différents.

De même, les moyens de génération de flux d'air peuvent être communs à l'ensemble des logements ou au contraire être individuels en comprenant un moyen de génération de flux d'air affecté à chaque logement. Selon la divulgation les moyens de génération de flux d'air peuvent aussi être uniquement raccordés à la chambre de mélange lorsque le dispositif de diffusion selon l'invention en possède une.

Selon une forme de réalisation les moyens de génération de flux d'air comprennent des premiers moyens raccordés aux moyens d'admissions des logements. La position des moyens de génération de flux d'air en amont de la cartouche par rapport au sens de circulation du flux d'air évite tout dépôt de composition odorante sur les moyens de génération de flux d'air.

Selon une variante de cette forme de réalisation, les premiers moyens de génération de flux d'air comprennent pour chaque logement un pulseur d'air individuel piloté par l'unité de commande. La mise en œuvre de pulseurs d'air individuels permet de moduler simplement le flux d'air dans chaque logement et donc la quantité de composition olfactive délivré par chaque logement.

Selon une autre caractéristique de la divulgation les moyens de génération de flux d'air comprennent des deuxièmes moyens directement raccordés à la chambre de mélange.

Selon une variante de cette caractéristique, les deuxièmes moyens de génération de flux d'air sont raccordés à la chambre de mélange en amont du raccordement des moyens d'échappement des logements à la chambre de mélange. La mise en œuvre de deuxièmes moyens de génération de flux d'air directement raccordés à la chambre de mélange permet d'obtenir au niveau de la bouche de diffusion un débit d'air supérieur à la somme des débits d'air issu des logements ce qui permet d'effectuer un meilleur mélange. De plus, la position en amont des deuxièmes moyens de génération de flux d'air évite leur encrassement ou pollution par les compositions olfactives.

Selon une autre variante de cette caractéristique, les deuxièmes moyens de génération de flux d'air comprennent un pulseur d'air raccordé à la chambre de mélange au niveau de l'extrémité opposée à la bouche de diffusion. La mise en œuvre d'un tel pulseur d'air permet d'engendrer un flux turbulent favorable au mélange des compositions odorantes dans la chambre.

Selon une autre caractéristique de la divulgation le dispositif comprend des moyens de lecture de moyens d'identification présents sur les cartouches et l'unité de commande est apte à modifier le mode de fonctionnement du dispositif en fonction des lectures. Selon une autre caractéristique de la divulgation chaque logement présente une forme en partie au moins asymétrique destinée à coopérer avec une cartouche de forme complémentaire de manière à définir un sens d'introduction de ladite cartouche. Cette caractéristique permet d'assurer un détrompage et de guider un utilisateur en ce qui concerne le sens d'introduction de la cartouche dans son logement.

Bien entendu, les différentes caractéristiques, variantes et formes de réalisation d'une cartouche et d'un dispositif de diffusion selon la divulgation peuvent être associées les unes avec les autres selon diverses combinaisons dans la mesure où elles ne sont pas incompatibles ou exclusives les unes des autres.

Par ailleurs, diverses autres caractéristiques de l'invention ressortent de la description annexée effectuée en référence aux dessins qui illustrent une forme non limitative de réalisation d'une cartouche et d'un dispositif de diffusion
- La figure 1 est une vue perspective schématique d'une cartouche
- La figure 2 une coupe axiale de la cartouche de la figure 1 avec les moyens d'obturation de cette dernière en position fermée,
- La figure 3 est une vue éclatée d'un bouchon constitutif de la cartouche de la figure 1,
- La figure 4 une coupe axiale analogue à la figure 2 avec les moyens d'obturation de la cartouche en position ouverte,
- La figure 5 est une section schématique d'une partie centrale de la cartouche illustrée figure 1,
- La figure 6 est une perspective schématique d'un diffuseur
- La figure 7 est autre perspective schématique du diffuseur illustré figure 6 avec un tiroir ouvert,
- La figure 8 est une perspective schématique partielle arrachée du diffuseur illustré figure 6 montrant des détails constructifs de ce dernier,
- La figure 9 est une vue de gauche de la perspective de la figure 8
- La figure 10 est une coupe selon le plan XI-XI de la figure 9,
- La figure 11 est une vue d'une variante d'une cartouche avec les moyens d'obturation de cette dernière en position fermée,
- La figure 12 est une vue de la cartouche de la figure 11 avec les moyens d'obturation en position ouverte,
- La figure 13 est une vue d'une autre variante d'une cartouche avec les moyens d'obturation de cette dernière en position fermée,
- La figure 14 est une vue de la cartouche de la figure 13 avec les moyens d'obturation en position ouverte.

Il est à noter que sur ces figures les éléments structurels et/ou fonctionnels communs aux différentes variantes peuvent présenter les mêmes références.

L'invention vise à permettre la diffusion de fragrances résultant notamment du mélange à la demande de compositions odorantes. A cette effet, l'invention propose la mise en œuvre d'un dispositif de diffusion dans lequel sont disposées au moins deux et de préférence plus de deux cartouches amovibles contenant chacune une composition odorante. Le dispositif de diffusion selon l'invention est en outre adapté pour contrôler la circulation d'air dans les cartouches pour assurer une diffusion sèche des compositions odorantes qu'elles contiennent.

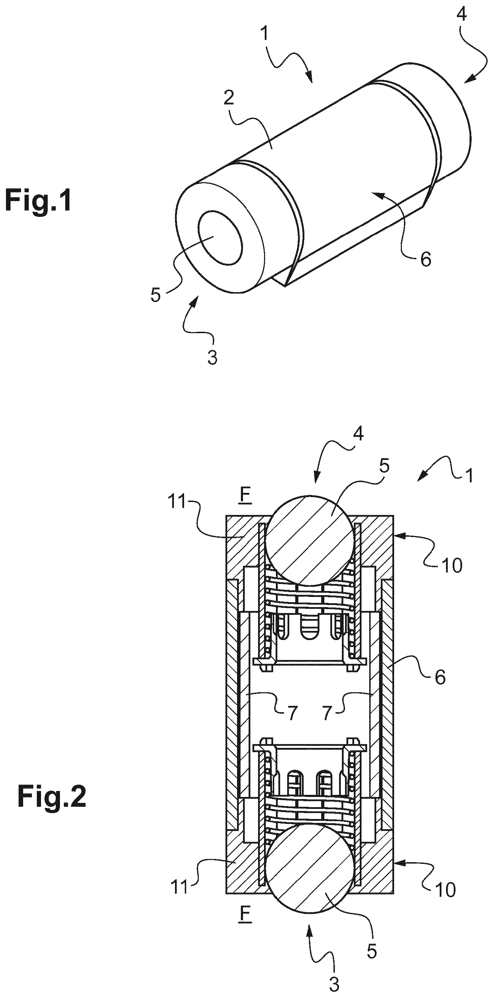

Une cartouche selon la divulgation telle qu'illustrée à la figure 1 et désignée dans son ensemble par la référence 1, comprend un corps creux 2 pourvu d'une entrée 3 et d'une sortie 4 chacune associée à des moyens d'obturations 5 asservis en fermeture comme cela ressort de la suite.

Selon la forme de réalisation présentée et comme le montre la figure 2, le corps 2 possède une partie centrale 6 tubulaire de forme générale cylindrique à l'intérieur de laquelle sont disposés des moyens d'accumulation 7 d'une composition odorante. Selon l'exemple illustré, les moyens d'accumulation 7 sont réalisés sous la forme d'une chemise en matériaux absorbant tel que le matériau commercialisé sous la marque POREX ^{®}. Les moyens d'accumulation 7 définissent alors un canal central pour la circulation d'un flux d'air dans la cartouche 1. Les moyens d'accumulation 7 forment donc un réservoir de composition odorante adaptés pour une diffusion sèche. Les moyens d'accumulation 7 sont, de préférence mais non nécessairement, chargés en composition odorante préalablement à leur mise en place dans la cartouche. En variante, les moyens d'accumulation 7 peuvent avoir d'autres formes, par exemple, une forme rectangulaire traversante de la cartouche, de manière à créer au moins deux canaux de circulation du flux d'air autour de cette forme. Toute autre configuration peut également être envisagée. Ainsi, les moyens d'accumulation peuvent être réalisés sous la forme d'un ensemble de billes absorbantes disposées en vrac dans la cartouche.

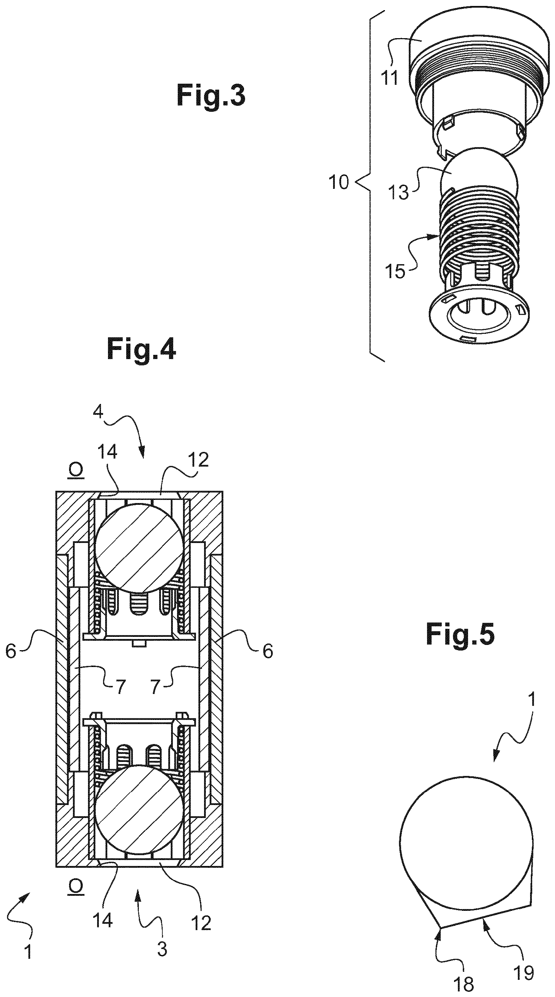

Le corps 2 de la cartouche comprend en outre deux bouchons 10 qui ferment la partie centrale 6 en immobilisant les moyens d'accumulation 7 à l'intérieur de cette dernière et qui définissent respectivement l'entrée 3 et la sortie 4 de la cartouche. Comme cela apparaît plus particulièrement à la figure 3, chaque bouchon 10 comprend un élément de liaison 11 adapté sur la partie centrale 6 de manière étanche à l'air. L'élément de liaison 11 comprend en outre un passage 12, visible à la figure 4, formant l'entrée 3 ou la sortie 4 de la cartouche 1. Afin d'assurer une obturation étanche à l'air du passage 12, le bouchon 10 comprend un obturateur 13 mobile en translation entre une position F de fermeture du passage 12 et une position O d'ouverture du passage 12. Dans le cas présent l'obturateur 13 est réalisé sous la forme d'une bille destinée à venir en appui sur un siège tronconique 14 formant le passage 12. Le bouchon 10 comprend également des moyens d'asservissement en fermeture 15 de l'obturateur 13. Dans le cas présent les moyens asservissements en fermeture 15 sont réalisés sous la forme d'un ressort hélicoïdal. Chaque bouchon 10 présente alors une configuration pouvant s'apparenter à un clapet anti retour, les deux bouchons étant montés en opposition sur la partie centrale 6 du corps de la cartouche.

Selon l'exemple illustré, le corps 2 de la cartouche 1 présente localement une asymétrie de révolution externe comme le montre la figure 5. Dans le cas présent, cette asymétrie locale est constituée par un méplat 18 s'étendant le long de la partie centrale 6 et supportant des moyens d'identification 19 de la cartouche adaptés pour une lecture automatique. Les moyens d'identification 19 sont, par exemple, constitués par une puce RFID.

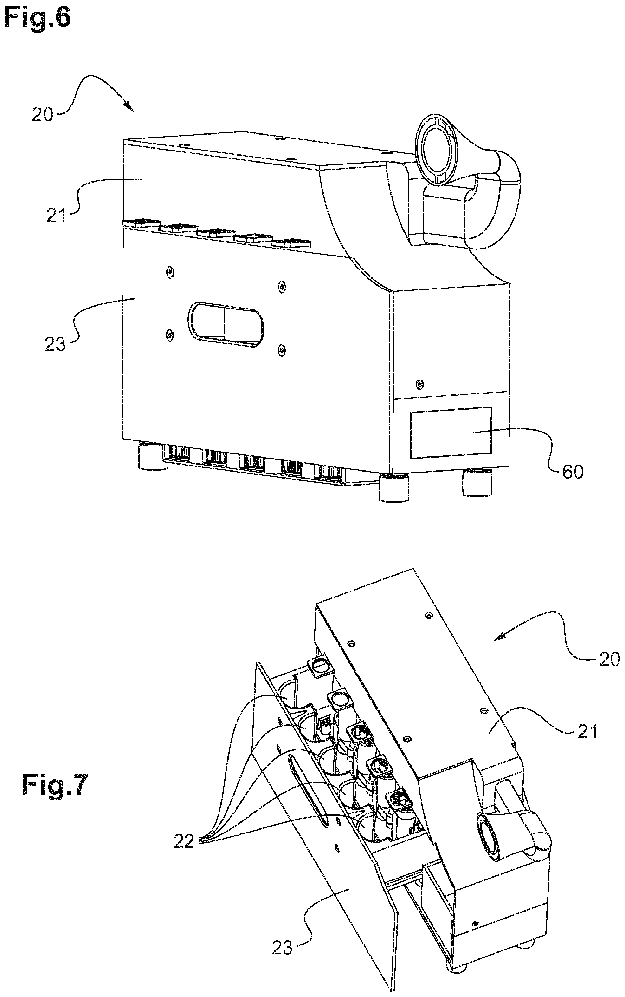

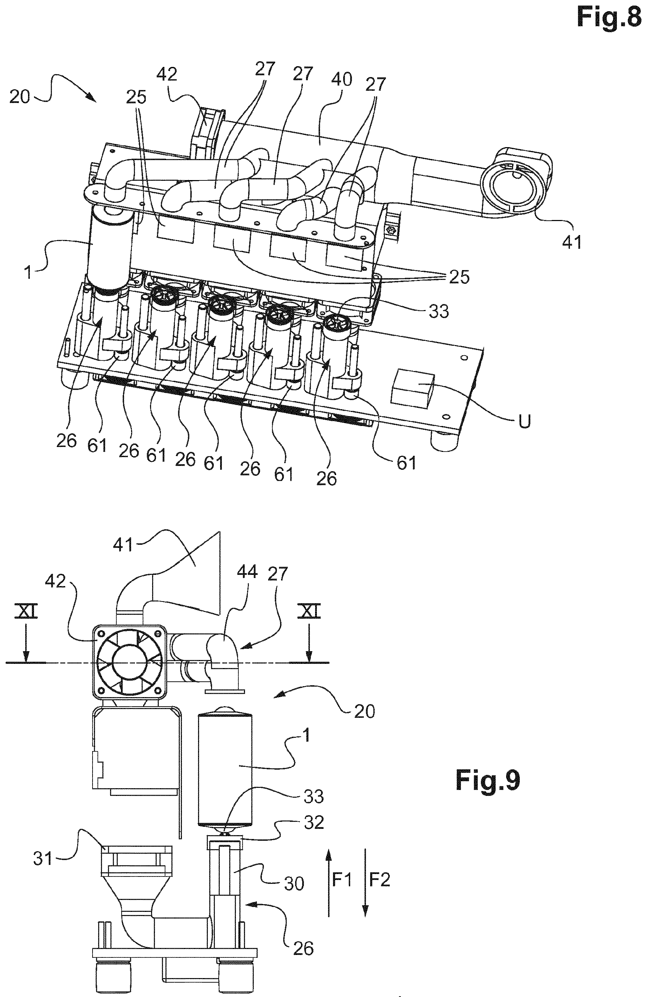

Une cartouche, selon la divulgation telle qu'ainsi constituée, est destinée à être utilisée dans un dispositif de diffusion 20, tel que représenté aux figures 6 et 7. Selon l'exemple illustré, le dispositif de diffusion 20 comprend un boitier 21 susceptible de recevoir cinq cartouches amovibles 1 et comprend, à cet effet, cinq logements 22 aménagés dans un tiroir 23 comme le montre la figure 7. Chaque logement 22 présente une forme complémentaire de celle de la cartouche 1 et, dans le cas présent, plus particulièrement de sa partie centrale 6. L'asymétrie de la cartouche et du logement de forme complémentaire assure alors une fonction de détrompeur garantissant une bonne mise en place de ladite cartouche de manière que les moyens d'identification 19 sont disposés en regard de moyens de lecture 25, visibles figure 8, équipant le dispositif de diffusion 20 et raccordés à une unité de commande U de ce dernier.

Comme le montrent les figures 8 et 9, chaque logement 22, destiné à recevoir une cartouche 1, comprend des moyens d'admission 26 et des moyens d'échappement 27 d'un flux d'air

Les moyens d'admission 26 sont destinés à être raccordés à l'entrée 3 de la cartouche 1 correspondante et adaptés pour agir sur les moyens d'obturations 5 correspondants. Dans le cas présent, les moyens d'admissions 26 associés à chaque logement 22 comprennent un circuit d'admission 30 dont une extrémité est raccordée à des premiers moyens de génération de flux d'air 31. L'autre extrémité du circuit d'admission comprend une tête de raccordement 32 pourvu d'un élément de manœuvre 33 formant un doigt apte à pousser l'obturateur de l'entrée de la cartouche 1.

Selon l'exemple illustré, les premiers moyens de génération de flux d'air 31 sont formés par cinq pulseurs d'air ou ventilateurs, chacun affecté à un logement 22. Les ventilateurs 31 sont en outre pilotés par l'unité de commande U adaptée pour contrôler la vitesse de rotation des ventilateurs 31 et donc leur débit.

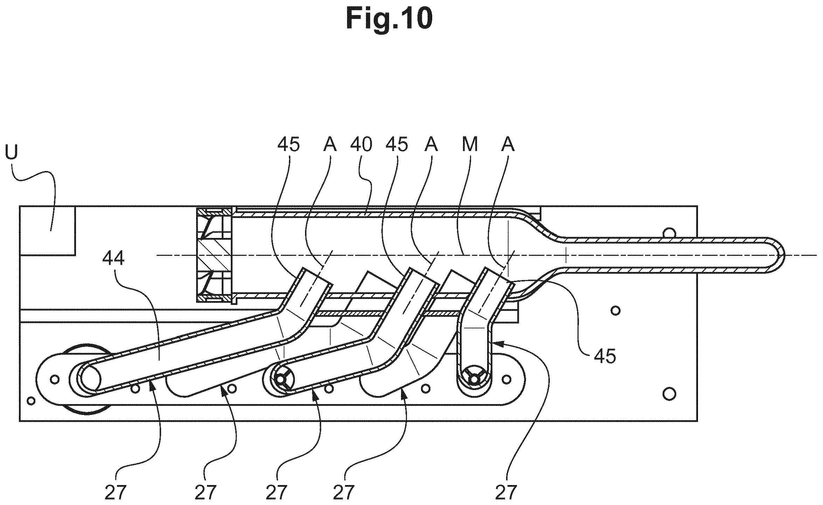

Les moyens d'échappement 27 sont destinés à être raccordés à la sortie 4 de la cartouche 1 correspondante et adaptés pour agir sur les moyens d'obturations 5 correspondants et sont raccordés à une chambre de mélange 40 comme le montre la figure 10.

Selon l'exemple illustré, la chambre de mélange 40 possède une forme allongée s'étendant le long d'une fibre moyenne M. La chambre de mélange 40 s'ouvre vers l'extérieur au niveau d'une de ses extrémités par une bouche de diffusion 41 tandis que son autre extrémité est raccordée à des deuxièmes moyens de génération de flux d'air 42. Dans le cas présent, les moyens de génération de flux 42 sont formés par un pulseur d'air ou ventilateur piloté par l'unité de commande U adaptée pour en contrôler le débit.

Les moyens d'échappement 27 comprennent, pour chaque logement 22, un circuit d'échappement 44 dont une extrémité est située au niveau du logement 22, en étant sensiblement en regard de la tête 32 du circuit d'admission 30 correspondant. L'extrémité du circuit d'échappement 44 située au niveau du logement 22 présente une tête de raccordement, non visible sur les figures, analogue à la tête 32 du circuit d'admission 30 et adaptée comme cette dernière pour agir sur les moyens d'obturations 5 de la sortie 4 d'une cartouche 1 située dans le logement 22 correspondant et permettre leur ouverture.

L'autre extrémité 45 du circuit d'échappement 44 s'étend jusqu'à la chambre de mélange 40 en faisant saillie dans celle-ci. Il est à noter que le tronçon du circuit d'échappement 44 s'étendant en saillie dans la chambre de mélange 40 possède un axe moyen A qui forme un angle aigu inférieur à 90° avec la fibre moyenne M de la chambre de mélange 40. Cette disposition limite les reflux dans le circuit d'échappement 34 et garantit la qualité du mélange réalisé dans la chambre 40. De plus, l'extension en saillie permet d'engendrer des turbulences également favorables à la qualité du mélange. En variante, d'autres moyens de mélange peuvent être envisageable, par exemple un agitateur tel qu'une lame ayant un mouvement rotatif au sein de la chambre ou encore une lame fixe agissant en déflecteur.

Le diffuseur de fragrance selon la divulgation est mis en œuvre de la manière suivante.

Tout d'abord, le tiroir 23 du dispositif 20 est ouvert pour permettre un accès aux logements 22. L'utilisateur peut alors facilement placer une cartouche 1 dans chaque logement 22. Le tiroir 23 est ensuite refermé de sorte que le dispositif se trouve à l'arrêt dans une configuration d'attente avant mise en marche. Il est à noter que dans cet état arrêté l'ensemble des cartouches placées dans les logements sont fermées par leurs moyens d'obturations.

Ensuite, l'utilisateur met en marche le diffuseur en agissant sur une interface 60 de l'unité de commande. Selon l'exemple illustré, l'interface est formée par un écran tactile relié à l'unité de commande. Toutefois, l'interface peut être réalisée de toute manière appropriée comme par exemple sous la forme de commande analogique ou encore sous la forme d'une interface radio, par exemple Wifi ou Bluetooth, permettant d'interagir avec l'unité de commande au moyen d'un ordinateur, un téléphone intelligent ou une tablette/ordinateur.

Lors de la mise en fonctionnement du dispositif de diffusion, l'unité de commande procède à la lecture des moyens d'identification des cartouches de manière à récupérer les données de ces dernières et notamment les informations relatives aux compositions odorantes contenues dans chacune des cartouches.

L'unité de commande détermine alors un mode de fonctionnement sur la base de l'identification des cartouches et de programmes préenregistrés ou alors sur la base d'instruction de l'utilisateur saisies sur l'interface. Pour les besoins de la description, il va être considéré que le mode de fonctionnement retenu vise à assurer la diffusion d'une fragrance composée du mélange des compositions odorantes de toutes les cartouches présentent dans le diffuseur.

Pour se faire, l'unité de commande pilote l'ouverture des moyens d'obturation de chacune des cartouches. A cet effet, l'unité de commande pilote des actionneurs 61 qui déplace en translation, dans le sens de la flèche F1, les moyens d'admission 26 vers l'intérieur du logement jusqu'à ce que l'entrée de chaque cartouche soit plaquée contre la tête 32 du circuit d'admission et la sortie de chaque cartouche soit plaquée contre la tête du circuit d'échappement 44. Ainsi les doigts 33 repoussent les moyens d'obturation correspondants de sorte que les entrées et les sorties des cartouches sont ouvertes. Il est à noter que le plaquage des cartouches contre les têtes des circuits d'admission et d'échappement assure un raccordement étanche à l'air entre les circuits et les cartouches. A cet effet, les têtes peuvent comprendre des joints d'étanchéités.

Pour assurer la diffusion de fragrance, l'unité de commande U pilote le fonctionnement des premiers et deuxièmes moyens de génération de flux d'air. Ainsi, les flux d'air injectés dans les cartouches s'y chargent en composition odorante et se mélangent dans la chambre 40. La modulation de la contribution de chacune des compositions odorantes au résultat final est pilotée par l'unité de commande U qui définit la vitesse de rotation des ventilateurs assignés à chacun des logements et donc le débit d'air dans ceux-ci. Par ailleurs, l'unité de commande U pilote la vitesse de rotation du ventilateur constitutif des deuxièmes moyens de génération de flux d'air ce qui permet notamment de moduler le temps de séjour des compositions odorantes dans la chambre de mélange.

Il est à noter que lorsque qu'une composition odorante ne doit pas contribuer à la fragrance diffusée, l'unité de commande U ne pilote pas l'actionneur 61 correspondant de manière à ne pas ouvrir ladite cartouche ou si cette dernière était en position ouverte l'unité de commande U pilote le fonctionnement de l'actionneur 61 de manière à déplacer les moyens d'admission dans le sens de la flèche F2. La cartouche se trouve ainsi désengagée des moyens de manœuvre des obturateurs intégrés aux circuit d'admission et d'échappement. Il est ainsi assuré une fermeture automatique de ladite cartouche.

De même, lorsque le diffuseur n'est pas utilisé l'unité de commande U pilote les actionneurs 61 de manière que toutes les cartouches soient désengagées et donc fermées. Il est à noter que l'unité de commande est adaptée pour permettre une diffusion séquentielle des compositions odorantes des cartouches, c'est-à-dire la mise en œuvre des cartouches les unes après les autres.

Selon l'exemple illustré et décrit précédemment, les moyens de manœuvre des obturateurs des cartouches sont passifs. Toutefois ces moyens de manœuvre pourraient être réalisés d'une autre façon et par exemple constitués par des doigts motorisés pilotés par l'unité de commande agissant chacun sur un obturateur.

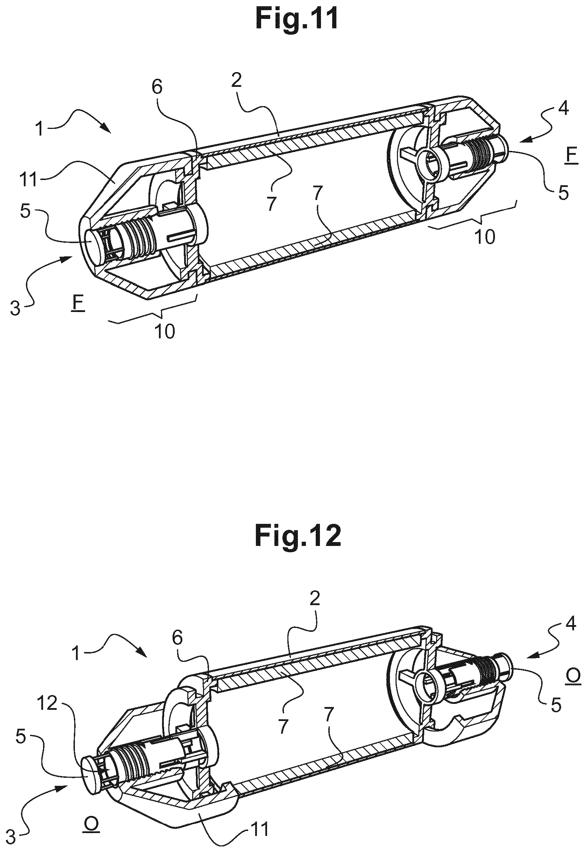

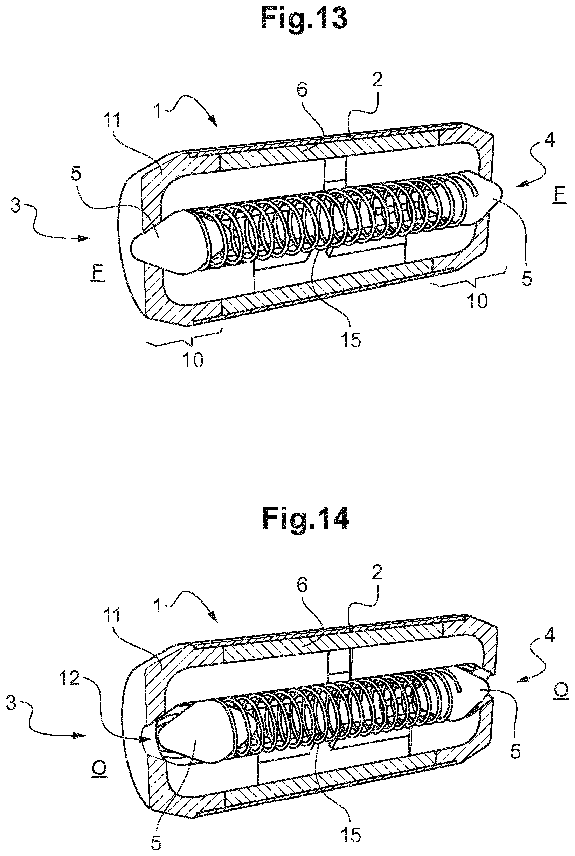

Les figures 11 à 14 illustrent des variantes de réalisation de la cartouche 1 Ces cartouches 1 sont analogues à la première version décrite. Sur la figure 11, le corps 2 de la cartouche comprend deux bouchons 10 qui ferment la partie centrale 6 en immobilisant les moyens d'accumulation 7 à l'intérieur de cette dernière et qui définissent respectivement l'entrée 3 et la sortie 4 de la cartouche. Chaque bouchon 10 comprend un élément de liaison 11 adapté sur la partie centrale 6 de manière étanche à l'air. L'élément de liaison 11 comprend en outre un passage 12, visible à la figure 12, formant l'entrée 3 ou la sortie 4 de la cartouche 1. Le bouchon 10 comprend un outre un obturateur 13 ayant la forme d'un disque mobile en translation vers l'extérieur de la cartouche. A la figure 13, les obturateurs 13, à chaque extrémité, présentent une forme allongée arrondie, et un unique ressort 15 les pousse contre l'ouverture afin de maintenir la cartouche en position fermée F. Des moyens d'admission 26 et d'échappement 27 du flux d'air viennent appuyer contre les obturateurs 5 de manière à placer la cartouche en position ouverte, visible à la figure 14.

Bien entendu, diverses autres variantes de réalisation des cartouches mais également du diffuseur peuvent être envisagées dans le cadre des revendications annexées.

## Revendications

1. Dispositif (20) pour la diffusion de fragrances, comprenant un boîtier (21) comportant au moins deux logements (22), chaque logement (22) étant adapté pour recevoir une cartouche (1) de manière amovible, la cartouche comprenant :
- un corps creux (2) pourvu d'une entrée (3) et d'une sortie (4) ;
- de moyens d'accumulation (7) d'au moins une composition odorante, les moyens d'accumulation étant disposés dans le corps creux de la cartouche ; et
- de moyens d'obturation (5) de l'entrée et de la sortie de la cartouche, les moyens d'obturation (5) étant mobiles entre une position d'ouverture (O) et une position de fermeture (F), les moyens d'obturation (5) étant asservis en fermeture et étanches à l'air en position de fermeture ;
chaque logement (22) du boîtier comprenant des moyens d'admission (26) et des moyens d'échappement (27) d'un flux d'air, destinés à être raccordés respectivement à l'entrée (3) et à la sortie (4) d'une cartouche (1) disposée dans le logement (22),
lequel dispositif (20) comprend des moyens de génération de flux d'air (31), adaptés pour générer des flux d'air traversant les logements (22) entre les moyens d'admission (26) et les moyens d'échappement (27),
**caractérisé en ce que** les moyens d'admission (26) et les moyens d'échappement (27) sont adaptés pour agir sur les moyens d'obturation (5) d'une cartouche, située dans un logement (22), de manière à déplacer les moyens d'obturation en position d'ouverture (O),
**et en ce que** le dispositif comprend une unité de commande (U) apte à contrôler les moyens d'admission (26), les moyens d'échappement (27) et les moyens de génération de flux d'air (31), pour assurer une diffusion indépendante, séquentielle ou simultanée, dans des proportions variables, des compositions odorantes contenues dans les cartouches, et une fermeture des cartouches lors de l'arrêt de l'appareil,
**et en ce que** les moyens d'admissions (26) comprennent un circuit d'admission (30) dont une extrémité est raccordée aux premiers moyens de génération de flux d'air (31), et l'autre extrémité comprend une tête de raccordement (32) pourvue d'un élément de manœuvre (33) formant un doigt apte à pousser les moyens d'obturation (5) de l'entrée d'une cartouche (1) présente dans un logement (22), lesquels premiers moyens de génération de flux d'air (31) comprennent, pour chaque logement (22), un pulseur d'air individuel piloté par l'unité de commande (U), lesquels moyens de génération de flux d'air (31) comprennent des premiers moyens raccordés aux moyens d'admissions des logements,
**et en ce que** les moyens d'échappement (27) comprennent un circuit d'échappement (44) dont une extrémité est raccordée à la chambre de mélange (40), et l'autre extrémité comprend une tête de raccordement adaptée pour agir sur les moyens d'obturation (5) de la sortie de la cartouche (1) présente dans le logement (22),
**et en ce que** le dispositif comprend une chambre de mélange (40) qui s'ouvre vers l'extérieur par une bouche de diffusion (41) et à laquelle les moyens d'échappement (27) de chacun des logements (22) sont raccordés en amont de la bouche de diffusion (41), lesquels moyens de génération de flux d'air comprennent des deuxièmes moyens directement raccordés à la chambre de mélange (40), lesquels deuxièmes moyens de génération de flux d'air comprennent un pulseur d'air raccordé à la chambre de mélange au niveau de l'extrémité opposée à la bouche de diffusion (41).

2. Dispositif selon la revendication 1, **caractérisé en ce que** la chambre de mélange (40) présente une forme allongée le long d'une fibre moyenne (M), et **en ce que** la bouche de diffusion (41) se trouvant à une extrémité de la chambre de mélange, et **en ce que** les moyens d'échappement (27) sont raccordés à la chambre de mélange (40) par des tubulures dont l'axe moyen forme avec la fibre moyenne (M) de la chambre de mélange, un angle aigu inférieur à 90 degrés convergeant en direction de la bouche de diffusion.

3. Dispositif selon la revendication 2, **caractérisé en ce que** les tubulures de raccordement s'étendent en saillie à l'intérieur de la chambre de mélange.

4. Dispositif selon l'une des revendications 1 à 3, **caractérisé en ce que** les deuxièmes moyens de génération de flux d'air sont raccordés à la chambre de mélange, en amont du raccordement des moyens d'échappement des logements à la chambre de mélange.

5. Cartouche (1), pour un dispositif de diffusion de fragrances selon l'une des revendications 1 à 4, comprenant :
- des moyens d'accumulation (7) d'au moins une composition odorante, disposés dans un corps creux (2), le corps creux étant pourvu d'une entrée (3) et d'une sortie (4) obturées par un bouchon (10),
- chaque bouchon (10) comprenant :
- un élément de liaison (11) adapté sur le corps creux (2) de la cartouche de manière à ce que le bouchon soit étanche à l'air,
- un siège (14) définissant un passage (12) à travers le bouchon (10),
- un obturateur (5) mobile se déplaçant dans le corps creux, entre une position d'ouverture (0) et une position de fermeture (F) du passage (12),
- des moyens d'asservissement (15) de l'obturateur en position de fermeture, de sorte que l'obturateur (5) obture le passage (12) du bouchon de façon étanche à l'air,
**caractérisée en ce que** les moyens d'asservissement comprennent un organe élastique (15).

6. Cartouche selon la revendication 5, **caractérisé en ce que** les moyens d'asservissement en fermeture de l'obturateur comprennent un ressort.

7. Cartouche selon la revendication 6, **caractérisé en ce que** les moyens d'asservissement comprennent un ressort à lame ou un ressort hélicoïdal.

8. Cartouche selon l'une des revendications 5 à 7, **caractérisée en ce que** l'obturateur est réalisé sous la forme d'une bille (13) destinée à venir en appui sur un siège tronconique (14) formant le passage (12).

## Patentansprüche

1. Vorrichtung (20) zum Verbreiten von Düften mit einem mindestens zwei Abteile (22) aufweisenden Gehäuse (21), wobei jedes Abteil (22) dazu ausgelegt ist, eine Kartusche (1) in herausnehmbarer Weise aufzunehmen, wobei die Kartusche
- einen mit einem Eingang (3) und einem Ausgang (4) versehenen Hohlkörper (2),
- Mittel (7) zum Speichern mindestens einer duftenden Mischung, wobei die Speichermittel (7) in dem Hohlkörper der Kartusche angeordnet sind, und
- Mittel (5) zum Verschließen des Eingangs und des Ausgangs der Kartusche, wobei die Verschlußmittel (5) zwischen einer Öffnungsposition (O) und einer Verschlußposition (F) bewegbar sind, wobei die Verschlußmittel (5) zum Verschließen geregelt werden und in der Verschlußposition luftdicht sind,
aufweist;
wobei jedes Abteil (22) des Gehäuses Zulaufmittel (26) und Entweichungsmittel (27) für einen Luftstrom aufweist, die dazu bestimmt sind, an den Eingang (3) beziehungsweise an den Ausgang (4) einer in das Abteil (22) eingesetzten Kartusche (1) angeschlossen zu werden,
wobei die Vorrichtung (20) Mittel (31) zum Erzeugen von Luftströmen aufweist, die dazu ausgelegt sind, Luftströme zu erzeugen, die die Abteile (22) zwischen den Zulaufmitteln (26) und den Entweichungsmitteln (27) durchqueren,
**dadurch gekennzeichnet, daß** die Zulaufmittel (26) und die Entweichungsmittel (27) dazu ausgelegt sind, auf die Mittel (5) zum Verschließen einer in einem Abteil (22) befindlichen Kartusche einzuwirken, um die Verschlußmittel in die Öffnungsposition (O) zu bringen,
**und daß** die Vorrichtung eine Steuereinheit (U) aufweist, die geeignet ist, die Zulaufmittel (26), die Entweichungsmittel (27) und die Mittel (31) zum Erzeugen von Luftströmen zu steuern, um ein unabhängiges, aufeinanderfolgendes oder gleichzeitiges Spenden der in den Kartuschen enthaltenen Duftstoffmischungen sicherzustellen und um ein Schließen der Kartuschen beim Abschalten des Geräts sicherzustellen,
**und daß** die Zulaufmittel (26) einen Zulaufkreis (30) aufweisen, von dem ein Ende mit den ersten Mitteln (31) zum Erzeugen von Luftströmen verbunden ist, und daß das andere Ende einen Anschlußkopf (32) aufweist, der mit einem Betätigungselement (33) versehen ist, das einen Finger bildet, der geeignet ist, die Verschlußmittel (5) des Eingangs einer in einem Abteil (22) vorhandenen Kartusche (1) zu schieben, wobei die ersten Mittel (31) zum Erzeugen von Luftströmen für jedes Abteil (22) ein durch die Steuereinheit (U) gesteuertes individuelles Luftgebläse aufweisen, wobei die Mittel (31) zum Erzeugen von Luftströmen erste mit den Zulaufmitteln der Abteile verbundene Mittel aufweisen,
**und daß** die Entweichungsmittel (27) einen Entweichungskreis (44) aufweisen, von dem ein Ende mit der Mischkammer (40) verbunden ist und daß das andere Ende einen Anschlußkopf aufweist, der geeignet ist, auf die Verschlußmittel (5) des Ausgangs der in dem Abteil (22) vorhandenen Kartusche (1) einzuwirken,
**und daß** die Vorrichtung eine Mischkammer (40) aufweist, die sich nach außen hin durch eine Spendermündung (41) öffnet, und mit der die Entweichungsmittel (27) jedes der Abteile (22) stromaufwärts der Spendermündung (41) verbunden sind, wobei die Mittel zum Erzeugen von Luftströmen mit der Mischkammer (40) direkt verbundene zweite Mittel aufweisen, wobei die zweiten Mittel zum Erzeugen von Luftströmen ein mit der Mischkammer in Höhe des der Spendermündung (41) entgegengesetzten Endes verbundenes Luftgebläse aufweisen.

2. Vorrichtung gemäß Anspruch 1, **dadurch gekennzeichnet, daß** die Mischkammer (40) eine sich entlang einer Mittelfaser (M) erstreckende Form aufweist und daß sich die Spendermündung (41) an einem Ende der Mischkammer befindet und daß die Entweichungsmittel (27) durch Röhren an die Mischkammer (40) angeschlossen sind, deren Mittelachse mit der Mittelfaser (M) der Mischkammer einen spitzen Winkel von weniger als 90° bildet, der zur Spendermündung hin konvergiert.

3. Vorrichtung gemäß Anspruch 2, **dadurch gekennzeichnet, daß** sich die Anschlußröhren innerhalb der Mischkammer hervorstehend erstrecken.

4. Vorrichtung gemäß einem der Ansprüche 1 bis 3, **dadurch gekennzeichnet, daß** die zweiten Mittel zum Erzeugen von Luftströmen an die Mischkammer angeschlossen sind, und zwar stromaufwärts vom Anschluß der Entweichungsmittel der Kammern an die Mischkammer.

5. Kartusche (1) für eine Vorrichtung zum Verbreiten von Düften gemäß einem der Ansprüche 1 bis 4 mit
- Mitteln (7) zum Speichern mindestens einer duftenden Mischung, die in einem Hohlkörper (2) angeordnet sind, wobei der Hohlkörper mit einem Eingang (3) und einem Ausgang (4), die durch einen Stopfen (10) verschlossen sind, versehen ist,
- wobei jeder Stopfen (10)
- ein Verbindungselement (11), das an den Hohlkörper (2) der Kartusche so angepaßt ist, daß der Stopfen luftdicht ist,
- einen Sitz (14), der einen Durchlaß (12) durch den Stopfen (10) definiert,
- einen beweglichen Verschluß (5), der sich im Hohlkörper zwischen einer Öffnungsposition (O) und einer Verschlußposition (F) des Durchlasses (12) bewegt,
- Mittel (15) zum Steuern des Verschlusses in die Verschlußposition derart, daß der Verschluß (2) den Durchlaß (12) des Stopfens luftdicht verschließt,
aufweist,
**dadurch gekennzeichnet, daß** die Steuerungsmittel ein elastisches Organ (15) aufweisen.

6. Kartusche gemäß Anspruch 5, **dadurch gekennzeichnet, daß** die Mittel zum Steuern des Verschlusses in die Verschlußposition eine Feder aufweisen.

7. Kartusche gemäß Anspruch 6, **dadurch gekennzeichnet, daß** die Steuerungsmittel eine Blattfeder oder eine Spiralfeder aufweisen.

8. Vorrichtung gemäß einem der Ansprüche 5 bis 7, **dadurch gekennzeichnet, daß** der Verschluß in Form einer Kugel (13) ausgebildet ist, die dazu bestimmt ist, auf einem den Durchlaß (12) bildenden konischen Sitz (14) aufzuliegen.

## Claims

1. A fragrance dispensing device (20) comprising a casing (21) including at least two compartments (22), each compartment (22) being adapted to removably receive a cartridge (1), the cartridge comprising:
- a hollow body (2) provided with an inlet (3) and an outlet (4);
- accumulation means (7) for at least one odorant composition, the accumulation means being arranged in the hollow body of the cartridge; and
- shutting means (5) for the inlet and the outlet of the cartridge, the shutting means (5) being mobile between an open position (O) and a closed position (F), the shutting means (5) being closure-controlled and airtight in the closed position;
each compartment (22) of the casing comprising air-flow intake means (26) and exhaust means (27), intended to be connected respectively to the inlet (3) and to the outlet (4) of a cartridge (1) arranged in the compartment (22), which device (20) comprises air-flow generation means (31), adapted to generate air flows passing through the compartments (22) between the intake means (26) and the exhaust means (27), **characterized in that** the intake means (26) and the exhaust means (27) are adapted to act on the shutting means (5) of a cartridge, located in a compartment (22), so as to displace the shutting means to the open position (O),
**and in that** the device comprises a control unit (U) adapted to control the intake means (26), the exhaust means (27) and the air-flow generation means (31), so as to provide an independent, sequential or simultaneous diffusion, in variable proportions, of the odorant compositions contained in the cartridges, and a closure of the cartridges when the apparatus is stopped,
**and in that** the intake means (26) comprise an intake circuit (30) one end of which is connected to the first air-flow generation means (31), and the other end of which comprises a connection head (32) provided with an actuating element (33) forming a finger able to push the shutting means (5) of the inlet of a cartridge (1) present in a compartment (22), which first air-flow generation means (31) comprise, for each compartment (22), an individual air blower controlled by the control unit (U), which air-flow generation means (31) comprise first means connected to the intake means of the compartments,
**and in that** the exhaust means (27) comprise an exhaust circuit (44) one end of which is connected to the mixing chamber (40), and the other end of which comprises a connection head adapted to act on the shutting means (5) of the outlet of the cartridge (1) present in the compartment (22),
**and in that** the device comprises a mixing chamber (40) which opens to the outside through a diffusion mouth (41) and to which the exhaust means (27) of each of the compartments (22) are connected upstream from the diffusion mouth (41), which air-flow generation means comprise second means directly connected to the mixing chamber (40), which second air-flow generation means comprise an air blower connected to the mixing chamber at the end opposite to the diffusion mouth (41).

2. The device according to claim 1, **characterized in that** the mixing chamber (40) has an elongate shape along a mean fibre (M), and **in that** the diffusion mouth (41) is located at one end of the mixing chamber, and **in that** the exhaust means (27) are connected to the mixing chamber (40) by pipes whose mean axis forms, with the mean fibre (M) of the mixing chamber, an acute angle lower than 90 degrees, converging towards the diffusion mouth.

3. The device according to claim 2, **characterized in that** the connection pipes extend in protrusion inside the mixing chamber.

4. The device according to one of claims 1 to 3, **characterized in that** the second air-flow generation means are connected to the mixing chamber, upstream from the connection of the exhaust means of the compartments to the mixing chamber.

5. A cartridge (1), for a fragrance dispensing device according to one of claims 1 to 4, comprising:
- accumulation means (7) for at least one odorant composition, arranged in a hollow body (2), the hollow body being provided with an inlet (3) and an outlet (4) shut by a plug (10),
- each plug (10) comprising:
- a link element (11) fitted on the hollow body (2) of the cartridge in such a way that the plug is airtight,
- a seat (14) defining a passage (12) through the plug (10),
- a shutter (5) which is mobile and moves in the hollow body, between an open position (O) and a closed position (F) of the passage (12),
- closure-control means (15) for the shutter in the closed position, so that the shutter (5) shuts the passage (12) of the plug in an airtight manner,
**characterized in that** the closure-control means comprise an elastic member (15).

6. The cartridge according to claim 5, **characterized in that** the closure-control means for the shutter comprise a spring.

7. The cartridge according to claim 6, **characterized in that** the closure-control means comprise a leaf spring or a helical spring.

8. The cartridge according to one of claims 5 to 7, **characterized in that** the shutter is in the form of a ball (13) intended to bear against a frustoconical seat (14) forming the passage (12).
